# EUROPEAN PATENT APPLICATION

(11) **EP 4 461 792 A1**
(43) Date of publication of application: **13.11.2024**
(21) Application number: 23461577.1
(22) Date of filing: 07.05.2023
(51) Int. Cl.: C11B 9/02

(54) **THE METHOD FOR OBTAINING ESSENTIAL OIL FROM CURCUMA LONGA L., MICROBIOLOGICAL MEDIUM WITH TURMERIC ESSENTIAL OIL FOR YEASTS AND MOLDS AND A METHOD FOR OBTAINING A MICROBIOLOGICAL MEDIUM**

(71) Applicant: Uniwersytet Zielonogórski, 65-417 Zielona Góra (PL)
(72) Inventor: Kliks, Jaroslaw, 66-120 Kargowa (PL); Korycka-Korwek, Justyna, 62-128 Zielona Gora (PL); Mrowczynska, Maria, 62-273 Zielona Gora (PL); Krolicka, Zuzanna, 66-100 Sulechow (PL)
(74) Representative: JD&P Patent Attorneys Joanna Dargiewicz & Partners

(57) **Abstract**

The subject of the present invention is a method for obtaining essential oil from *Curcuma longa L.,* comprising the steps in which: shredded curcuma rhizome *Curcuma longa L.* subjected to the freezing process at -75°C; the lyophilization process is carried out using the following parameters: 24 h, temperature 20°C, pressure 200Pa, 48 h, temperature 32°C, pressure 20Pa, condenser temperature -50°C; the effluent collected from the lyophilizer condenser is centrifuged at 4000 RPM for 5-10 minutes, preferably for 8 minutes; the resulting supernatant is separated into a non-polar phase - essential oil and an aqueous phase; drying the separated essential oil, preferably in the presence of anhydrous sodium VI sulfate. The invention also relates to an essential oil-containing medium, a method for its preparation and use.

## Description

The subject of the present invention is a method for obtaining essential oil from *Curcuma longa L.* with bacteriostatic properties, a microbiological medium with turmeric essential oil for the growth of yeasts and molds and a method for producing a microbiological medium with turmeric essential oil for the growth of yeasts and molds.

The growing interest in natural components with health-promoting properties causes the search for new substances in commonly used herbs and spices. Turmeric oil has many uses in both health and beauty care. Its small amount is enough to take advantage of the benefits of turmeric. It also has an advantage over the powdered spice itself due to the higher concentration of active ingredients in the oil, thanks to which a small amount is enough to obtain the same concentrations of biologically active compounds in the blood. Active compounds found in turmeric oil have strong antioxidant and anti-inflammatory properties.

About 3.5-5.5% of the essential oil is obtained from the turmeric rhizome. Depending on the type of oil, the parameters of optical rotation and refractive index differ. In addition, the composition varies even within the same species grown in different regions. Oil obtained from Curcuma long *turmeric longa L.* it is classified in the fourth degree of toxicity, which proves its low negative impact on the human body, thanks to which it is safe when used in a microbiological medium, e.g. for pregnant women working in a microbiological diagnostic laboratory, unlike the standard chloramphenicol.

DG18 microbiological medium (Dichloran Glycerol Agar), i.e. a selective medium with low water activity for the determination of xerophilic forms from dried and semi-dried food products, as well as a general-purpose medium for the counting of yeasts and molds in food products, contains an antibiotic that inhibits the growth bacteria - chloramphenicol, which unfortunately has a toxic effect on the human body. The use of this ingredient makes the medium not 100% safe for the employees preparing it in the laboratory.

Chinese patent application CN110915651A discloses Zedoray medium *(Curcuma zedoaria)* for tissue culture. Zedoray tissue culture medium contains the following ingredients: 8 g lactose, 5 g bovine bile salt, 0.2 mg 6-benzylaminopurine, 0.4 mg indole butyric acid; 30 g of sucrose, 6 g of agar powder, 5 g of sodium chloride and 1000 ml of water. The preparation of the Zedoray tissue culture medium includes the following steps: mixing the ingredients except lactose, bovine bile salt, 6-benzylaminopurine, indolebutyric acid, sucrose and agar powder, heating to dissolve the ingredients, and adjusting the pH so that after sterilization the pH is 5.8 +/- 0.2; adding agar powder, 6-benzylaminopurine and indolebutyric acid and heating until dissolved; adding other ingredients, shaking until uniformly mixed to obtain a mixture, and filling separately; followed by high pressure steam sterilization at 121°C for 15 minutes, cooling to 60°C and pouring the Zedoray tissue culture medium mixture onto the plates.

US patent application US2020016508A1 describes an exemplary method for extracting phytochemical oil from plant parts which comprises freezing plant parts from at least one *Cannabis plant sativa, Curcuma longa, Panax ginseng* and *Panax quinquefolius* to obtain at least one of the pure, uncontaminated cannabis oils, turmeric oil and ginseng oil, respectively, depending on which plants were selected for the process. With *Cannabis sativa* is obtained from cannabis oil, from *Curcuma longa* turmeric oil, and *Panax ginseng* or *P. quinquefolius* is a good source of ginseng oil.

Chinese patent application CN110664966A discloses a method for processing zedoara turmeric .

Korean patent application KR20110069975A relates to a pharmaceutical composition for the prevention or treatment of prostatic hyperplasia, which contains *Curcuma aromatica* extract as an active ingredient *Curcuma aromatica* extract is prepared by extracting *Curcuma aromatica* with 60-90% ethanol.

Korean patent application KR20120040504A discloses a method for producing fermented long curcuma using *Lactobacillus johnsonii* IOCC-9203.

The aim of the invention was to develop a method of producing a microbiological medium for the quantitative determination of yeasts and molds in food products, based on turmeric oil obtained at low temperatures, in the lyophilization process, as well as a method of obtaining turmeric oil in the lyophilization process.

This goal was achieved by the method of obtaining essential oil from *Curcuma longa L.* with bacteriostatic properties, a microbiological medium with turmeric essential oil for the growth of yeasts and molds and a method for producing a microbiological medium with turmeric essential oil for the growth of yeasts and molds according to the present invention.

Thus, the present invention provides a method for obtaining Curcuma essential oil *longa L.,* including the stages in which: shredded curcuma rhizome *Curcuma longa L.* subjected to the freezing process at -75°C; the lyophilization process is carried out using the following parameters: 24 h, temperature 20°C, pressure 200Pa, 48 h, temperature 32°C, pressure 20Pa, condenser temperature -50°C; the effluent collected from the lyophilizer condenser is centrifuged at 4000 RPM for 5-10 minutes, preferably for 8 minutes; the resulting supernatant is separated into a non-polar phase - essential oil and an aqueous phase; drying the separated essential oil, preferably in the presence of anhydrous sodium VI sulfate. The invention also relates to an essential oil-containing medium, a method for its preparation and use.

Preferably, essential oils are extracted from *Curcuma longa L.* turmeric rhizome by a vacuum drying process. They are preferably combined with the recipe of a selective microbiological medium for the isolation and quantitative determination of yeasts and molds in food samples.

### EXAMPLES

### Example 1 - obtaining essential oil, vacuum drying of turmeric rhizomes

The choice of the method of extracting the essential oil from the plant is important because it has a decisive influence on its properties. High temperature can negatively affect the chemical compounds present in the oil, and solvent extraction can leave small amounts of it in the final product.

The raw material, *Curcuma longa L.* turmeric rhizome, was used to obtain the essential oil.

The turmeric was ground using a knife grinder. Then, the freeze-drying process was carried out, in which the shredded turmeric rhizome was first subjected to a freezing process at - 75°C. Then, the freeze-drying process was carried out taking into account the following parameters:
- 24 h, temperature 20°C, pressure 200Pa,
- 48 h, temperature 32°C, pressure 20Pa,
- condenser temperature -50°C.

The effluent collected from the lyophilizer condenser was centrifuged at 4000 RPM for 8 minutes, then the obtained supernatant was transferred to the separator, the non-polar phase - essential oil was separated from the water phase. The separated oil was dried in the presence of anhydrous sodium VI sulfate.

The obtained oil (concentration 3.6%) contains a significant share of volatile compounds, which significantly differed from oils obtained using high-temperature methods.

### Example 2 - preparation of medium with turmeric essential oil for selective enumeration of yeasts and molds in food products

Turmeric essential oil was selected as a component of a microbiological medium for the quantification of yeasts and molds in food products. A dry base medium was formulated to be dissolved in water and sterilized at 121°C for 15 minutes (Table 1) and a substrate supplement to impart selectivity to the medium (Table 2).

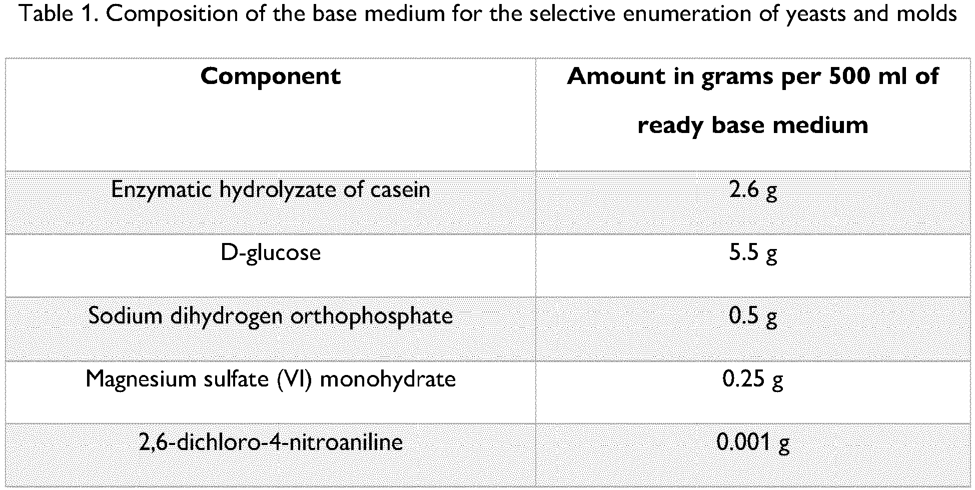

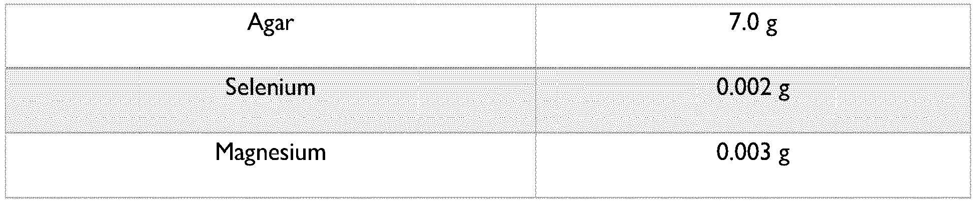

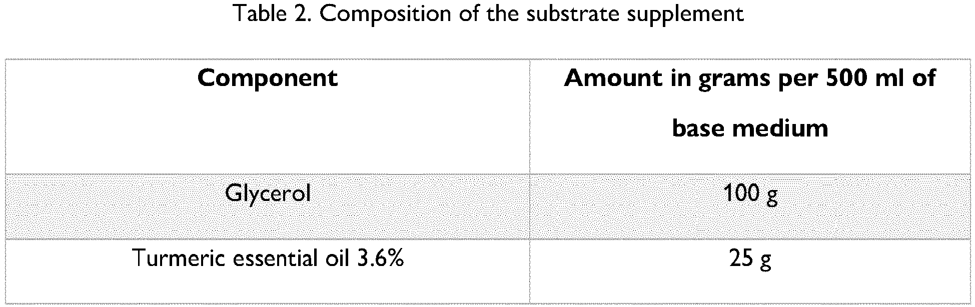

A loose base medium of pale yellow color was obtained. 15.25 g of the obtained base medium was dissolved in 500 cm³ of distilled water, thoroughly mixed and heated until complete dissolution. Then, 125 g of the supplement was added to the dissolved medium. The mixture was autoclaved at 121°C for 15 minutes. After the sterilization process, the pH was adjusted (at 25°C) to the value of 5.6 ± 0.2. The medium thus obtained (straw color) was poured into sterile Petri dishes. The ready substrate was stored at 2-8°C, and the loose, dehydrated substrate at 10-30°C.

### Example 3 - use of turmeric essential oil medium for the determination of yeasts and molds

The following were used for comparative research:
- test material - microbiological medium with turmeric essential oil obtained in Example 2;
- reference material - medium Dichloran Glycerol Agar Base (DG-18).

The DG-18 substrate was prepared in accordance with the manufacturer's instructions, and the substrate tested as described in Example 2. The research matrix - Balton bread with visible signs of mold inside the crumb was used for microbiological inoculation.

The sample for microbiological cultures was prepared by homogenization in buffered peptone water in the amount of: 10 g of the sample and 90 g of sterile buffered peptone water. Then, decimal dilutions of the sample were made and the prepared dilutions were plated on Petri dishes with prepared media. The samples were incubated at 25°C for 5 days.

Three replicates were performed for each trial. After the incubation period, the number of microorganisms on the plates was read (Table 3).

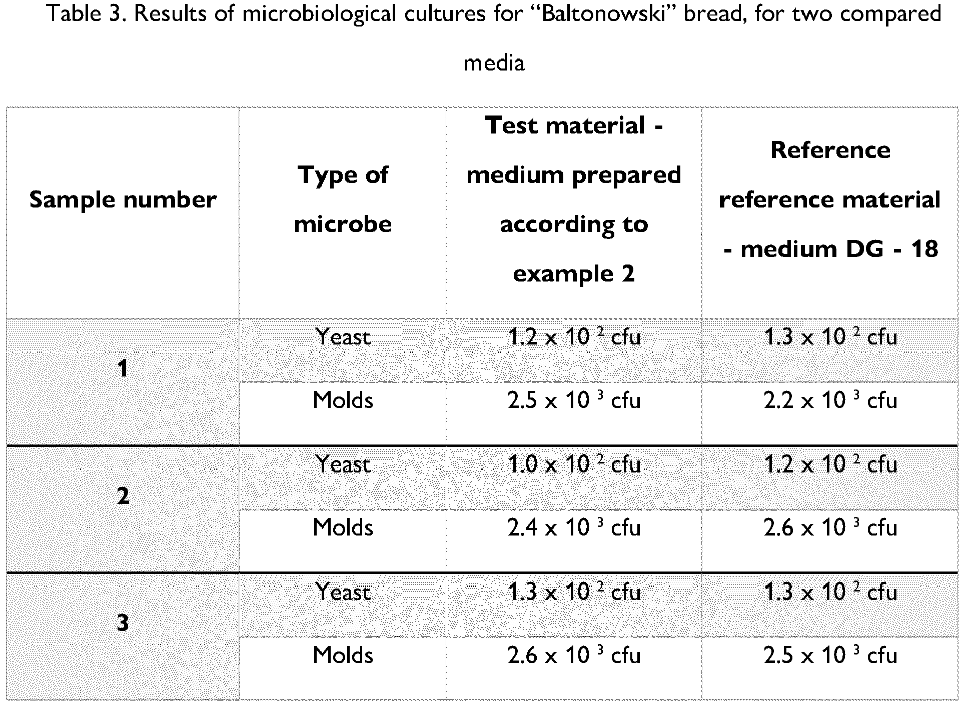

Comparable results of microbiological cultures were observed on both test and reference media. Mold and yeast colonies on both media were equally readable. The incubation time for counting the colonies was 5 days for both media. No growth of bacterial colonies was observed on the media, which proves their selectivity and inhibitory (for microorganisms other than yeasts and moulds) nature of the ingredients used in the supplements of both media, i.e. in the reference reference medium - chloramphenicol, and in the medium composed according to Example 2 - essential oil turmeric essential oil.

## Claims

1. A method of obtaining essential oil from *Curcuma longa L.,* comprising the steps in which:
- shredded curcuma rhizome *Curcuma longa L.* is subjected to a freezing process at -75°C;
- the lyophilization process is carried out using the following parameters:
24 h, temperature 20°C, pressure 200Pa,
48 h, temperature 32°C, pressure 20Pa,
condenser temperature -50°C;
- the effluent collected from the lyophilizer condenser is centrifuged at 4000 RPM for 5-10 minutes, preferably for 8 minutes;
- the obtained supernatant is separated into a non-polar phase - essential oil and a water phase;
- drying the separated essential oil, preferably in the presence of anhydrous sodium (VI) sulfate.

2. A microbiological medium containing a base medium and a substrate supplement with the following compositions:
The composition of the base medium 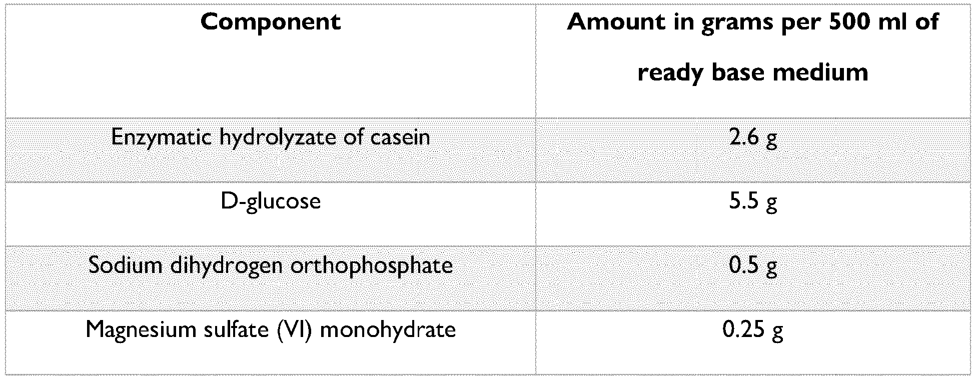 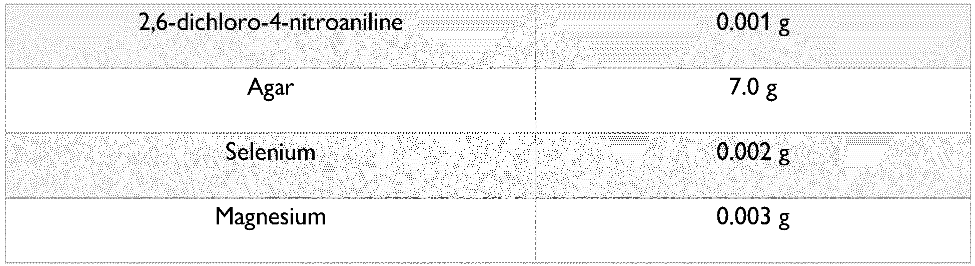
The composition of the substrate supplement

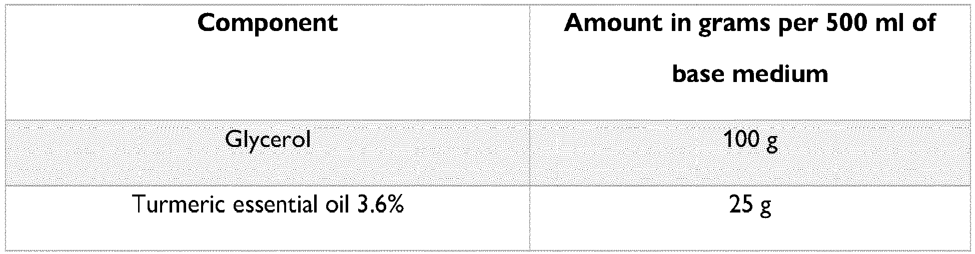

3. The microbiological medium according to claim 2, **characterized in that** it comprises turmeric essential oil obtained by the process as defined in claim 1.

4. Use of the microbiological medium according to claim 2 or 3 for the determination of yeasts and molds, preferably in food products.

5. A method for obtaining a microbiological medium containing a base medium and a substrate supplement with the following compositions:
The composition of the base medium 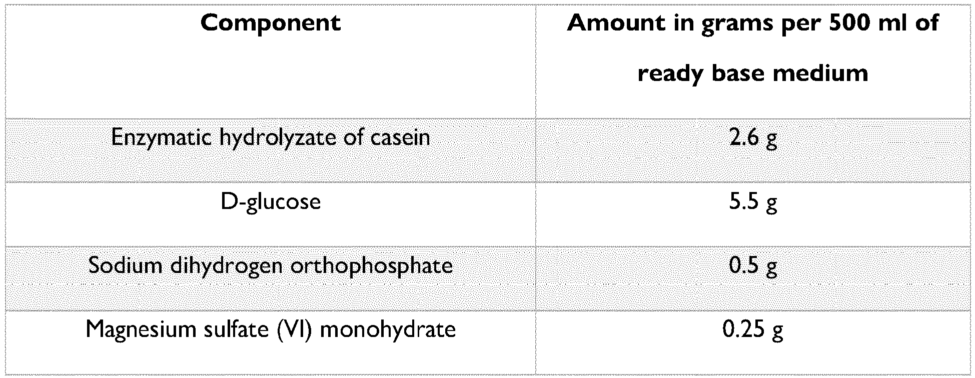 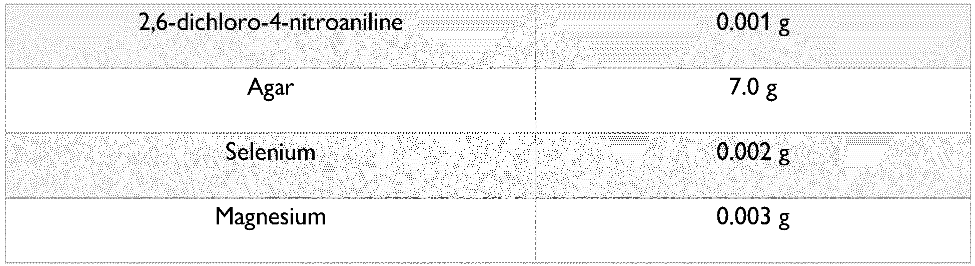
The composition of the substrate supplement 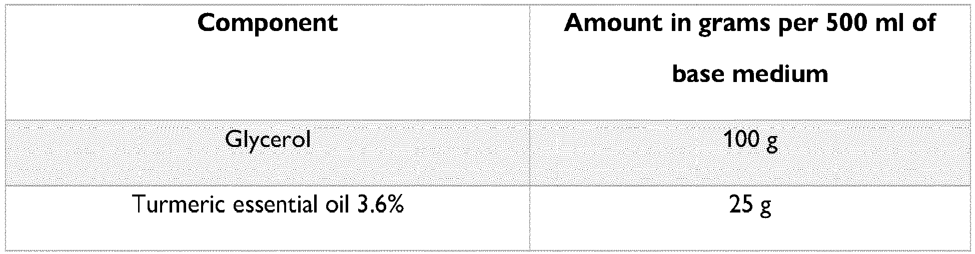
comprising the steps in which:
- 15.25 g of the obtained base medium is dissolved in 500 cm³ of distilled water, stirred and heated until complete dissolution;
- 125 g of substrate supplement is added to the dissolved base medium;
- the mixture is autoclaved at 121°C for 15 minutes;
- after cooling the mixture to 25°C, the pH is adjusted to 5.6 ± 0.2.
